# EUROPEAN PATENT SPECIFICATION

(11) **EP 2 797 573 B1**
(45) Date of publication and mention of the grant of the patent: **22.08.2018**
(21) Application number: 12810279.5
(22) Date of filing: 20.12.2012
(51) Int. Cl.: A61Q 5/02, A61K 8/41, A61K 8/44, A61K 8/46, A61Q 5/00, A61Q 5/12, A61Q 5/10

(54) **METHOD OF TREATING ARTIFICIALLY COLOURED HAIR**
VERFAHREN ZUR BEHANDLUNG VON KÜNSTLICH GEFÄRBTEM HAAR
PROCÉDÉ DE TRAITEMENT POUR CHEVEUX COLORÉS ARTIFICIELLEMENT

(30) Priority: 29.12.2011 EP 11196074
(43) Date of publication of application: 05.11.2014
(73) Proprietor: Kao Germany GmbH, 64297 Darmstadt (DE)
(72) Inventor: HOFFMANN, Martin, 64673 Zwingenberg (DE); OLSSON, Kristin, 68526 Ladenburg (DE)
(74) Representative: Grit, Mustafa
(86) International application number: PCT/EP2012/076482
(87) International publication number: WO 2013/098210

(56) References cited:
- EP-A1- 1 366 755
- EP-A1- 2 018 841
- EP-A1- 2 184 051
- EP-A1- 2 191 812
- EP-A1- 2 382 962
- WO-A1-2008/070566
- DE-C1- 19 735 865
- US-A- 6 143 286
- SUBIRATS N ET AL: "Shampoo for dyed hair: effect of surfactants and additives on colour fading due to washing process and UV light exposure", COMMUNICACIONES PRESENTADAS A LAS JORNADAS DE COMITE ESPAGNOLDE LA DETERGENCIA, BARCELONA, ES, vol. 38, 1 January 2008 (2008-01-01), pages 67-78, XP009121479, ISSN: 0212-7466
- DATABASE GNPD [Online] MINTEL; 31 July 2007 (2007-07-31), "Two-in-One Shampoo & Conditioner", Database accession no. 736946

## Description

Present invention relates to a method of treating artificially coloured hair with a cleansing composition wherein the composition comprises anionic and cationic foaming surfactants.

Coloring human hair is being practiced for ages. The dyestuffs used are so called direct dyes, having itself a colour, or oxidative dyes which do not have a colour but turns into coloured substances after oxidative polymerization. The aim of dyeing has not only been achieving nice colours it has also been to achieve long lasting colours. In achieving long lasting colours the dyestuffs used certainly play important role. However, the compositions used for cleansing and conditioning hair play also important role as depending on their composition they may wash out more or less dyestuff from hair.

It has been known in the field of hair cleansing that foaming surfactants used have a strong affinity to wash out dyestuffs from hair. Since the type of foaming surfactants believed to have different colour wash out effect, it has always been the first priority to use different kinds of foaming surfactant and/or vary the concentrations of the individual surfactants and/or use low total surfactants concentration. Despite all the efforts, the problem of colour wash-out from artificially colored hair has remained unsolved and therefore there is a great need for new solution in order to secure log lasting colours.

The aim of the present inventors have, therefore, been to provide new measures to secure less colour wash out from hair and therewith achieve long lasting colours. EP2 184 051 discloses hair cleansing compositions for reducing colour fading from artificially coloured hair, the compositions comprising anionic amino acid surfactants, alkyl ether sulphate surfactants and cationic polymers. The inventors have surprisingly found out that when an artificially coloured hair is treated with a cleansing composition as defined in claim 1, colour life is substantially extended.

Accordingly, the first object of the present invention is a method for treating artificially coloured hair wherein a cleansing composition comprising 5 to 50% by weight, calculated to the total composition, foaming surfactant, wherein the composition comprises at least one alkyl ether sulphate surfactant and at least one amino acid surfactant as anionic foaming surfactants and at least one cationic surfactant of the general structure 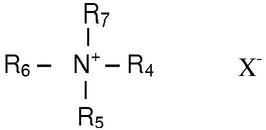 where R₄ is a saturated or unsaturated, branched or linear alkyl chain with 8-22 C atoms, R₅, R₆ and R₇ are H or a saturated or unsaturated, branched or linear alkyl chain with 1 to 4 C atoms or saturated or unsaturated, branched or linear C2 to C4 alkyl chain with one or more substituent, preferably a hydroxyl substituent and X is typically an anionic such as chloride, bromide and methosulfate, is applied onto hair and rinsed off from hair with water.

The second object of the present invention is the use of at least one cationic surfactant of the general structure 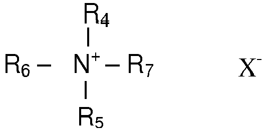 where R₄ is a saturated or unsaturated, branched or linear alkyl chain with 8-22 C atoms, R₅, R₆ and R₇ are H or a saturated or unsaturated, branched or linear alkyl chain with 1 to 4 C atoms or saturated or unsaturated, branched or linear C2 to C4 alkyl chain with one or more substituent, preferably a hydroxyl substituent and X is typically an anion such as chloride, bromide and methosulfate in a cleansing composition comprising 5 to 50% by weight, calculated to the total composition, foaming surfactant, wherein the composition comprises at least one alkyl ether sulphate surfactant and at least one amino acid surfactant as anionic foaming surfactants, for reducing colour wash out from artificially coloured hair.

The cleansing composition of the present invention used to treat artificially coloured hair is preferably provided in a kit comprising one or more compositions wherein one of the compositions is the cleansing composition used in the hair treatment method of the present invention. Preferably the kit comprises another composition comprising at least one hair dye and more preferably a third composition is comprised comprising at least one oxidizing agent.

The cleansing composition comprises 5 to 50%, preferably 10 to 30%, more preferably 10 to 25% by weight, calculated to the total composition, foaming surfactant. The composition comprises at least one alkyl ether sulphate surfactant and at least one amino acid surfactant as anionic foaming surfactants. Preferably, the composition further comprises nonionic surfactants and most preferably at least one nonionic and at least one amphoteric surfactants. The cleansing composition comprises at least one anionic surfactant and at least one nonionic surfactant preferably at a weight ratio in the range of 10:1 to 1:1, more preferably 7:1 to 2:1 and most preferably 5:1 to 3:1. In the same way anionic and amphoteric surfactants are comprises in a anionic to amphoteric surfactants weight ratio in the range of 10:1 to 1:1, more preferably 7:1 to 2:1 and most preferably 5:1 to 3:1.

The alkyl ether sulphate surfactants used as anionic surfactants within the meaning of the present invention are according to the general structure

R₁₁(OCH₂CH₂)ₙOSO₃⁻M⁺

wherein R₁₁ is a straight or branched, saturated or unsaturated alkyl chain with 10 to 24 C atoms, preferably 10 to 18 C atoms, more preferably 10 to 16 C atoms and most preferably 12 to 14 C atoms, n is a value between 1 and 5 and M is a cation such as ammonium, sodium, potassium, magnesium. Suitable and most preferred is sodium laureth sulphate available under the trade name Texapon from Cognis or Emal from Kao Corporation.

Additional anionic surfactants are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁₂-(C₂H₄O)ₙ-O-CH₂COOX,

wherein R₁₂ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 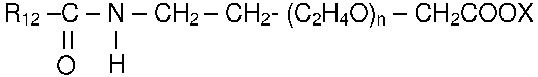 wherein R₁₂ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

Further anionic surfactants used within the meaning of the present invention are amino acid surfactants according to the general structure 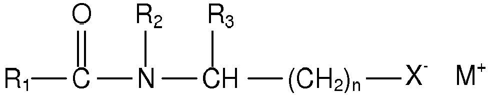 wherein R₁ is a saturated or unsaturated, straight or branched alkyl chain with 7 to 17 C atoms, preferably 9 to 17 C atoms, and more preferably 9 to 13 C atoms R₂ is H or a methyl, R₃ is H, COO⁻M⁺, CH₂COO⁻M or COOH, n is 0 to 2, X is COO⁻ or SO₃⁻ and M is independent from each other H, ammonium, sodium or potassium. It should be noted that alkyl chain includes also mixture of various alkyl chains as present especially in plant triglyceride derived alkyl chains such as cocoyl chain.

Suitably amino acid surfactant types are taurate, glutamate, alanin or alaninate, sarcosinate, aspartate surfactants, and mixtures thereof. Preferred are taurate, glutamate and sarcosinate surfactants and mixtures thereof. More preferred are sarcosinate and glutamates type surfactants and mixtures thereof.

Suitable taurate surfactants are according to the general formula 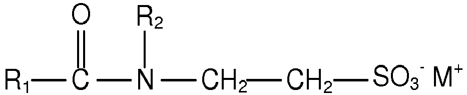 wherein R₁ is preferably a saturated or unsaturated, straight or branched alkyl chain with 7 to 17 C atoms, and more preferably 9 to 13 C atoms, R₂ is H or methyl, and M is H, sodium or potassium. Suitable examples are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof. Preferred are potassium cocoyl taurate, potassium methyl cocoyl taurate, sodium caproyl methyl taurate, sodium cocoyl taurate, sodium lauroyl taurate, sodium methyl cocoyl taurate and sodium methyl lauroyl taurate and mixtures thereof. More preferred are potassium cocoyl taurate, potassium methyl cocoyl taurate, sodium cocoyl taurate, sodium lauroyl taurate, sodium methyl cocoyl taurate and sodium methyl lauroyl taurate and mixtures thereof.

Suitable glutamate surfactants are according to the general formula 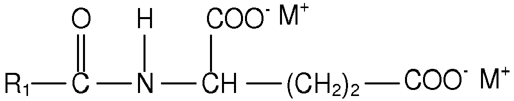 wherein R₁ is preferably a saturated or unsaturated, straight or branched alkyl chain with 7 to 17 C atoms, and more preferably 9 to 13 C atoms, and M is independent from each other H, ammonium, sodium or potassium. Suitable examples are dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, and sodium undecylenoyl glutamate and mixtures thereof. Preferred are disodium capryloyl glutamate, disodium cocoyl glutamate, disodium lauroyl glutamate, potassium capryloyl glutamate, potassium cocoyl glutamate, potassium lauroyl glutamate, potassium myristoyl glutamate, sodium capryloyl glutamate, sodium cocoyl glutamate, sodium lauroyl glutamate, and sodium myristoyl glutamate and mixtures thereof. More preferred are disodium cocoyl glutamate, disodium lauroyl glutamate, potassium cocoyl glutamate, potassium lauroyl glutamate, sodium cocoyl glutamate, and sodium lauroyl glutamate and mixtures thereof.

Suitable alanine or alaninate surfactants are according to the general formula 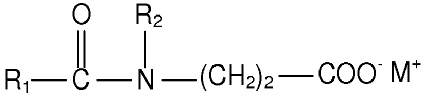 wherein R₁ is preferably a saturated or unsaturated, straight or branched alkyl chain with 7 to 17 C atoms, and more preferably 9 to 13 C atoms, R₂ is H or methyl and M is H, ammonium, sodium or potassium. Suitable examples are cocoyl methyl β-alanine, lauroyl β-alanine, lauroyl methyl β-alanine, myristoyl β-alanine, potassium lauroyl methyl β-alanine, sodium cocoyl alaninate, sodium cocoyl methyl β-alanine and sodium myristoyl methyl β-alanine and mixtures thereof.

Suitable glycine surfactants are according to the general formula 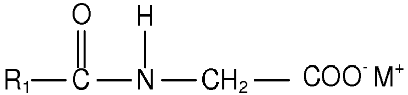 wherein R₁ is preferably a saturated or unsaturated, straight or branched alkyl chain with 7 to 17 C atoms, and more preferably 9 to 13 C atoms, and M is H, ammonium, sodium or potassium. Suitable examples are sodium palmitoyl glycine, sodium lauroyl glycine, sodium cocoyl glycine, sodium myristoyl glycine, potassium lauroyl glycine, and potassium cocoyl glycine and mixtures thereof.

Suitable sarcosinate surfactants are according to the general formula 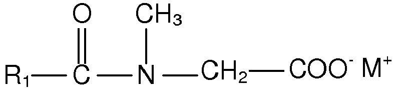 wherein R₁ is preferably a saturated or unsaturated, straight or branched alkyl chain with 7 to 17 C atoms, and more preferably 9 to 13 C atoms, and M is H, ammonium, sodium or potassium. Suitable examples are potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof. Preferred are ammonium lauroyl sarcosinate, ammonium cocoyl sarcosinate, potassium lauroyl sarcosinate, potassium cocoyl sarcosinate, sodium cocoyl sarcosinate, and sodium lauroyl sarcosinate and mixtures thereof. More preferred are sodium cocoyl sarcosinate, and sodium lauroyl sarcosinate and mixtures thereof.

Suitable aspartate surfactants are according to the general formula 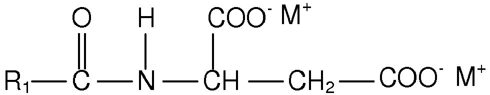 wherein R₁ is preferably a saturated or unsaturated, straight or branched alkyl chain with 7 to 17 C atoms, and more preferably 9 to 13 C atoms, and M is independent from each other H, ammonium, sodium or potassium. Suitable examples are sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof. Preferred are sodium lauroyl aspartate, sodium myristoyl aspartate, sodium cocoyl aspartate, and sodium caproyl aspartate and mixtures thereof.

It should be noted that compositions of the present invention can also comprise mixture of several type of amino acid surfactants such as mixture of glutamate and taurate surfactants, or mixture of taurate, glutamate and sarcosinate surfactants etc.

The preferred amino acid surfactants are glutamate and sarcosinate surfactants. It is also possible to use mixtures of several anionic surfactants, for example an ether sulphate, amino acid surfactant and a polyether carboxylic acid or alkyl amidoether carboxylic acid. The cleansing composition according to the present invention comprises at least one alkyl ether sulphate surfactant and at least one amino acid surfactant, especially glutamate and sarcosinate surfactants and in particular glutamate surfactant as anionic foaming surfactants.

In a preferred embodiment of the present invention, cleansing composition of the present invention comprises at least one alkyl ether sulphate surfactant and at least one amino acid surfactant as anionic surfactants as mentioned above and at least one nonionic surfactant.

Nonionic surfactants especially suited in the cleansing compositions according to the invention are alkyl polyglucosides of the general formula

R₁₃-O-(R₁₄O)ₙ-Zₓ,

wherein R₁₃ is an alkyl group with 8 to 18 carbon atoms, R₁₄ is an ethylene or propylene group, Z is a saccharide group with 5 to 6 carbon atoms, n is a number from 0 to 10 and x is a number between 1 and 5.

These alkyl polyglucosides are known in particular as excellent skin-compatible, foam improving agents in liquid detergents and body cleansing compositions. Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactants are, suitable for the cleansing compositions of the present invention, long-chain fatty acid dialkanolamides, such as coco fatty acid diethanolamide and myristic fatty acid diethanolamide.

Further suitable nonionic surfactants are amineoxides. Such amineoxides are state of the art, for example C₁₂-C₁₈-alkyl dimethyl amineoxides such as lauryl dimethyl amineoxide, C₁₂-C₁₈-alkyl amidopropyl or -ethyl amineoxides, C₁₂-C₁₈-alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in the compositions according to invention are C₁₀-C₂₂-fatty alcohol ethoxylate. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

Further nonionic surfactants are sorbitan surfactants suitable within the meaning of the present invention. Suitable non-limiting examples are esters of sorbitan such as sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan palmate, sorbitan sesquicaprylate, sorbitan sesquiisostearate, sorbitan sesquioleate, sorbitan sesquistearate and sorbitan stearate, as well as ethoxylated sorbitan surfactants such as PEG-20 sorbitan cocoate, PEG-40 sorbitan diisostearate, PEG-2 sorbitan isostearate, PEG-5 sorbitan isostearate, PEG-20 sorbitan isostearate, PEG-40 sorbitan laurate, PEG-10 sorbitan laurate, PEG-44 sorbitan laurate, PEG-75 sorbitan laurate, PEG-80 sorbitan laurate, PEG-3 sorbitan oleate, PEG-6 sorbitan oleate, PEG-20 sorbitan oleate, PEG-40 sorbitan oleate, PEG-80 sorbitan palmitate, PEG-3 sorbitan stearate, PEG-4 sorbitan stearate, PEG-6 sorbitan stearate, PEG-40 sorbitan stearate, and PEG-60 sorbitan stearate, and as well as Polysorbate 20, Polysorbate 21, Polysorbate 40, Polysorbate 60, Polysorbate 61, Polysorbate 65, Polysorbate 80, Polysorbate 81, and Polysorbate 85. Preferred are sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan palmate, sorbitan sesquicaprylate, sorbitan sesquiisostearate, sorbitan sesquioleate, sorbitan sesquistearate, sorbitan stearate, PEG-20 sorbitan cocoate, PEG-40 sorbitan diisostearate, PEG-2 sorbitan isostearate, PEG-5 sorbitan isostearate, PEG-20 sorbitan isostearate, PEG-40 sorbitan laurate, PEG-10 sorbitan laurate, PEG-44 sorbitan laurate, PEG-75 sorbitan laurate, PEG-80 sorbitan laurate, PEG-3 sorbitan oleate, PEG-6 sorbitan oleate, PEG-20 sorbitan oleate, PEG-40 sorbitan oleate, PEG-80 sorbitan palmitate, PEG-3 sorbitan stearate, PEG-4 sorbitan stearate, PEG-6 sorbitan stearate, PEG-40 sorbitan stearate, and PEG-60 sorbitan stearate.

The most preferred non-ionic surfactants are alkyl polyglucosides such as decyl, cocoyl polyglucoside and ethoxylated fatty alcohols such as laureth-16.

In a further preferred embodiment of the present invention, cleansing composition of the present invention comprises at least one alkyl ether sulphate surfactant and at least one amino acid surfactant as anionic surfactants, at least one nonionic surfactant and at least one amphoteric surfactant.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, suitable betaine surfactants are of general structure 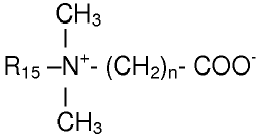 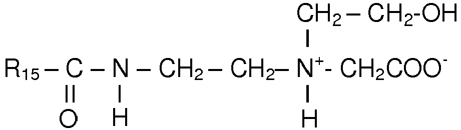
wherein R₁₅ is a C₈-C₁₈-alkyl group and n is 1 to 3;
sulfobetaines of the structure 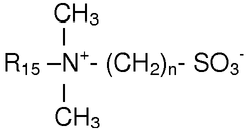
   wherein R₁₅ and n are same as above;
   hydroxysulfobetaines of the structure 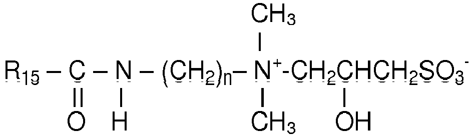
      wherein R₁₅ and n are same as above;
      and amidoalkyl betaines of the structure 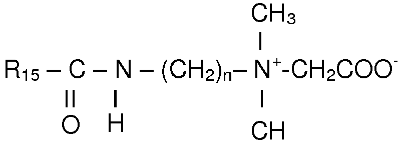 wherein R₁₅ and n are same as above.

The most preferred amphoteric surfactants are alkyl betaines such as lauryl betaine and alkyl amido betaines such as cocamidopropyl betaine and hydroxysulphobetaines such as cocamidopropyl hydroxysultaine.

In a particularly preferred embodiment of the present invention, aqueous cleansing composition comprises anionic surfactants of alkyl ether sulphate type and glutamate type of amino acid surfactants, at least one amphoteric surfactant especially alkyl amido alkyl betaine type and/or alkylamidoalkyl hydroxysultaine type and at least one non-ionic surfactant especially an alkyl polyglucoside type.

Compositions of the present invention comprise at least one cationic surfactant according to the above general structure. In principal any cationic surfactant according to the general structure is suitable for the purposes of the present invention. Suitable non-limiting examples to cationic surfactants are behentrimonium chloride, behentrimonium methosulphate, ceteartrimonium chloride, cetrimonium chloride, cetrimonium bromide, cetrimonium methosulphate, cocotrimonium chloride, cocotrimonium bromide, laurtrimonium chloride, laurtrimonium bromide, myrtrimonium chloride, steartrimonium chloride, steartrimonium chloride and soytrimonium chloride. Preferred are behentrimonium chloride, behentrimonium methosulphate, ceteartrimonium chloride, steartrimonium chloride, steartrimonium chloride, cetrimonium chloride, cetrimonium bromide and cetrimonium methosulphate. Most preferred are cetrimonium chloride, cetrimonium bromide and cetrimonium methosulphate.

Compositions of the present invention may certainly comprise mixture of the cationic surfactants.

Total concentration of cationic surfactant is in the range of 0.1 to 5%, preferably 0.2 to 4%, more preferably 0.25 to 3% and most preferably 0.5 to 2% by weight calculated to the total composition.

Aqueous cleansing composition of the present invention preferably comprises hair conditioning agents. Conditioning agents can be selected from oily substances, non-ionic substances and cationic polymers or their mixtures. It should be noted that cationic surfactants disclosed above may have conditioning effect on hair but are not meant as hair conditioning agents within the meaning of present invention.

Oily substances are selected from such as silicone oils, either volatile or nonvolatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule such as trimethyl pentaphenyl trisiloxane, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₉CO(OCH₂CH₂)ₙOH

or

R₁₉CO(OCH₂CH₂)ₙOOCR₂₀

where R₁₉ and R₂₀ are independent from each other saturated, unsaturated or branched or non-branched alkyl chain with 7 to 21 C atoms and n is typically 2 - 100.

In one of the preferred form of the present invention, aqueous cleansing compositions comprise at least one additional cationic polymer as a conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

Additionally cationic starch derived polymers especially oxidized cationic starch polymer known with it CTFA/INCI name hydroxypropyl Oxidized Starch PG-Trimonium chloride available from Graefe Chemie GmbH under the trade name Amylomer are comprised in the aquoues cleansing compositions of the present invention.

Furthermore, cationic polymers known with the CTFA/INCI adopted name Silicone Quaternium are also preferred cationic polymers. Expecially preferred is Silicone quaternium 22.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Total concentration of conditioning agents mentioned above namely oily substances, non-ionic substances and cationic polymers in the compositions is in the range of 0.01 to 5%, preferably 0.02 to 4%, more preferably 0.05 to 3% and most preferably 0.1 to 2.5% by weight calculated to total composition. Especially preferred are the cationic polymers as the conditioning agents in the given concentration ranges.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Aqueous cleansing composition may comprise at least one organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol and polypropylene glycols. Total concentration of organic solvents in the shampoo composition should not exceed 5% by weight, preferably in the range of 0.1 to 3%, more preferably 0.5 to 2.5% by weight calculated to total composition.

Aqueous cleansing composition of the present invention may further comprise at least one fatty alcohol of the following formula

R₂₁ - OH

wherein R₂₁ is straight or branched, saturated or unsaturated alkyl chain with 8 to 24, preferably 10 to 22, more preferably 12 to 18 and most preferably 12 to 16C atoms at a concentration of 0.1 to 5%, preferably 0.1 to 4% and more preferably 0.25 to 3% and most preferably 0.5 to 2.5% by weight calculated to total composition.

Suitable non-limiting preferred examples are decyl alcohol, myristyl alcohol, lauryl alcohol, cetyl alcohol, stearyl alcohol, behenyl alcohol, and arachidyl alcohol and their mixtures. More preferred are decyl alcohol, myristyl alcohol, lauryl alcohol, cetyl alcohol, and stearyl alcohol. Most preferred are decyl alcohol, myristyl alcohol and lauryl alcohol, and their mixtures.

Cleansing composition of the present invention may be pearly. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

The cleansing composition may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair and hair colour. In other words, anionic and non-ionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol^{R}". Extracts and the preparation thereof are also described in "Hagers Handbuch der pharmazeutischen Praxis", 4th Ed..

Compositions of the present invention may comprise further at least one compound according to the formula 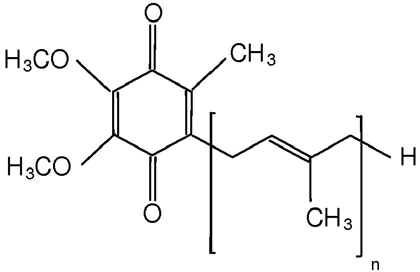 where n is a number from 1 to 10.

The compounds of the above formula are known as Ubiquinone, and also are known as Coenzyme. It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Aqueous cleansing compositions may further comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violet 2, Acid Red 33, Acid Orange 4, Acid Red 27 and Acid Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.001 to 5% by weight, preferably 0.01 to 4% more preferably 0.05 to 3% and most preferably 0.1 to 2.5% by weight calculated to total composition. The most preferred among the direct dyes is cationic direct dyes.

It is self-understood that the shampoos according to the invention may comprise other substances customarily used in such compositions such as preservatives, fragrances.

The pH of the compositions according to the present invention is suitably between 2 and 8.0, preferably in the range of 2.5 to 7.0, more preferably 3 to 6.5 and most preferably 4 to 5.5 measured at ambient temperature with a suitable pH meter.

pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Aqueous cleansing composition of the present invention preferably comprises one or more thickeners. Suitable ones are ethoxylated polyglyceryl esters with total ethoxy units in the range of 50 to 200 and fatty acyl chain length of 8 to 22 C atoms such as PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, and PEG-200 glyceryl tallowate, gylceryl oleate/cocoate and inorganic salt in particular sodium chloride when especially composition comprise alkyl ether sulphate type of surfactants.

Cleansing compositions of the present invention preferably has a viscosity in the range of 500 to 20,000 mPa.s, more preferably 1,000 to 15,000 mPa.s and most preferably 1,500 to 10,000 mPa.s measured at 20°C with a Brookfield viscosimeter using fro example Spindle 5 at appropriate rotation speed.

The following examples are to illustrate the invention, but not to limit. The compositions according to the invention are prepared by mixing the individual components in water, whereby it is also possible to use pre-mixtures of various ingredients.

### Example 1

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Cetrimonium chloride | 0.7 |
| Polyqauternium-10 | 0.5 |
| PEG-90 glyceryl isostearate | 3.0 |
| Citric acid/sodium hydroxide | q.s. to pH 5.5 |
| Preservative, fragrance | q.s |
| Water | to 100 |

The above shampoo was judged to have rich and creamy foam in a monadic test by the volunteers. It was furthermore mentioned that it foams very quickly. Additionally, the hair washed was excellently combable, had good shine, volume and body.

The above cleansing composition was tested against a composition not comprising cetrimonium chlrode chloride for its colour wash out effect. It was observed that the composition of Example 1 washed out much less colour that the composition not comprising the amino acid and cationic surfactant. It should be noted that the amino acid and cationic surfactants were replaced by anionic and non-ionic surfactants, respectively.

The colour was out test was carried out at 40°C using coloured hair tresses dipped into 5% solution of cleansing composition which were shaken at a speed of 50 pm. At predetermined times the tresses were taken out and after drying the colour was measured suing laboratory equipment and colour differences were calculated with well known method in the art.

Similar results were observed with the compositions below.

### Example 2

| | % by weight |
|---|---|
| Sodium lauryl ether sulphate | 10.6 |
| Cocamidopropyl hydroxysultaine | 3.0 |
| Sodium lauroyl sacosinate | 2.0 |
| Sodium lauroyl glutamate | 0.8 |
| Decyl glucoside | 2.0 |
| Silicone quaternium-22 | 0.2 |
| Steartrimonium chloride | 0.5 |
| Panthenol | 0.3 |
| PEG-90 glyceryl isostearate | 3.0 |
| Citric acid/sodium hydroxide | q.s. to pH 5.5 |
| Preservative, fragrance | q.s |
| Water | to 100 |

### Example 3

| | % by weight |
|---|---|
| Sodium lauryl ether sulphate | 10.6 |
| Cocamidopropyl betaine | 3.0 |
| Sodium lauroyl sarcosinate | 2.5 |
| Decyl glucoside | 2.0 |
| Polyquaternium-67 | 0.5 |
| Behentrimonium chloride | 0.5 |
| PEG-90 glyceryl isostearate | 3.0 |
| Lactic acid/sodium hydroxide | q.s. to pH 5.0 |
| Preservative, fragrance | q.s |
| Water | to 100 |

### Example 4

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Cetrimonium menthosulphate | 0.6 |
| Silicone quaternium-22 | 0.5 |
| PEG-90 glyceryl isostearate | 3.0 |
| Citric acid/sodium hydroxide | q.s. to pH 5.5 |
| Preservative, fragrance | q.s |
| Water | to 100 |

### Example 5

| | % by weight |
|---|---|
| Sodium lauryl ether sulphate | 10.6 |
| Cocamidopropyl hydroxysultaine | 3.0 |
| Sodium cocoyl sarcosinate | 3.0 |
| Sodium cocoyl glutamate | 0.8 |
| Laureth-16 | 2.0 |
| Cetrimonium chloride | 1.0 |
| Behenyl alcohol | 0.5 |
| Benzophenone-3 | 0.2 |
| PEG-90 glyceryl isostearate | 3.0 |
| Citric acid/sodium hydroxide | q.s. to pH 5.5 |
| Preservative, fragrance | q.s |
| Water | to 100 |

### Example 6

| | % by weight |
|---|---|
| Sodium lauryl ether sulphate | 10.6 |
| Cocamidopropyl hydroxysultaine | 3.0 |
| Sodium cocoyl glutamate | 0.5 |
| Sodium cocoly sarcosinate | 1.5 |
| Decyl glucoside | 2.0 |
| Cetrimonium chloride | 1.0 |
| Guar hydroxypropyltrimonium chloride | 0.5 |
| Benzophenone-3 | 0.2 |
| PEG-90 glyceryl isostearate | 3.0 |
| Basic red 51 | 0.1 |
| Basic orange 31 | 0.06 |
| Basic yellow 87 | 0.03 |
| Basic red 76 | 0.08 |
| Lactic acid/sodium hydroxide | q.s. to pH 5.5 |
| Preservative, fragrance | q.s |
| Water | to 100 |

The above shampoo gives hair additionally red shine.

## Claims

1. A method for treating artificially colored hair **characterized in that** a cleansing composition comprising 5 to 50% by weight, calculated to the total composition, of foaming surfactants, wherein the composition comprises at least one alkyl ether sulphate surfactant and at least one amino acid surfactant as anionic foaming surfactants and at least one cationic surfactant of the general structure 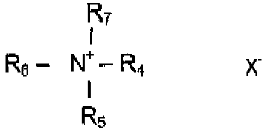 where R₄ is a saturated or unsaturated, branched or linear alkyl chain with 8-22 C atoms, R₅, R₆ and R₇ are H or a saturated or unsaturated, branched or linear alkyl chain with 1 to 4 C atoms or saturated or unsaturated, branched or linear C2 to C4 alkyl chain with one or more substituent, preferably a hydroxyl substituent and X is typically an anionic such as chloride, bromide and methosulfate is applied to the artificially coloured hair and rinsed off with water.

2. The method according to any of the preceding claims **characterized in that** cleansing composition comprises additionally nonionic foaming surfactants.

3. The method according to any of the preceding claims **characterized in that** cleansing composition comprises anionic, nonionic and amphoteric foaming surfactants.

4. The method according to any of the preceding claims **characterized in that** the anionic and nonionic surfactants are comprised in the cleansing composition at a weight ratio of anionic to nonionic surfactants in the range of 10:1 to 1:1.

5. The method according to any of the preceding claims **characterized in that** the anionic and amphoteric surfactants are comprised in the cleansing composition at a weight ratio of anionic to amphoteric surfactants in the range of 10:1 to 1:1.

6. The method according to any of the preceding claims **characterized in that** cleansing composition comprises alkyl polyglucoside and/or ethoxylated fatty alcohol as nonionic foaming surfactants.

7. The method according to any of the preceding claims **characterized in that** cleansing composition comprises alkyl betaines, alkyl amidobetaines and/or hydroxysulphobetaines as amphoteric foaming surfactants.

8. The method according to any of the preceding claims **characterized in that** cleansing composition comprises at least one cationic surfactant selected from behentrimonium chloride, behentrimonium methosulphate, ceteartrimonium chloride, cetrimonium chloride, cetrimonium bromide, cetrimonium methosulphate, cocotrimonium chloride, cocotrimonium bromide, laurtrimonium chloride, laurtrimonium bromide, myrtrimonium chloride, steartrimonium chloride, steartrimonium chloride and soytrimonium chloride.

9. The method according to any of the preceding claims **characterized in that** cleansing composition comprises at least one cationic surfactant at a concentration in the range of 0.1 to 5% by weight calculated to total composition.

10. The method according to any of the preceding claims **characterized in that** cleansing composition comprises at least one hair conditioning agent, preferably selected from cationic polymers.

11. The method according to any of the preceding claims **characterized in that** cleansing composition comprises one or more of the substances selected from organic solvents, fatty alcohols, pearlizing agents, solubilizers, UV filters, polyols, sequestering agents, natural plants extracts, ubiquinones, direct dyes and thickening agents.

12. Use of at least one cationic surfactant of the general structure 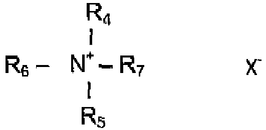 where R₄ is a saturated or unsaturated, branched or linear alkyl chain with 8-22 C atoms, R₅, R₆ and R₇ are H or a saturated or unsaturated, branched or linear alkyl chain with 1 to 4 C atoms or saturated or unsaturated, branched or linear C2 to C4 alkyl chain with one or more substituent, preferably a hydroxyl substituent and X is typically an anionic such as chloride, bromide and methosulfate in a cleansing composition comprising 5 to 50% by weight, calculated to the total composition, of foaming surfactant wherein the composition comprises at least one alkyl ether sulphate surfactant and at least one amino acid surfactant as anionic foaming surfactants, for reducing colour wash out from artificially coloured hair.

## Patentansprüche

1. Verfahren zum Behandeln künstlich gefärbter Haare, **dadurch gekennzeichnet, dass** eine Reinigungszusammensetzung, welche 5 Gewichts-% bis 50 Gewichts-% schaumbildende Tenside aufweist, bezogen auf die Gesamtzusammensetzung, wobei die Zusammensetzung mindestens ein Alkylethersulfat-Tensid und mindestens ein Aminosäure-Tensid als anionische schäumende Tenside und mindestens ein kationisches Tensid der allgemeinen Struktur 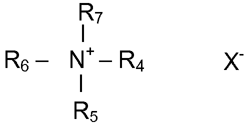 aufweist, wobei R₄ für eine gesättigte oder ungesättigte, verzweigte oder lineare Alkylkette mit 8 bis 22 C-Atomen steht, R₅, R₆ und R₇ für H oder eine gesättigte oder ungesättigte, verzweigte oder lineare Alkylkette mit 1 bis 4 C-Atomen oder eine gesättigte oder ungesättigte, verzweigte oder lineare C₂₋₄-Alkylkette mit einem oder mehreren Substituenten, vorzugsweise einem Hydroxy-Substituenten, steht und X typischerweise für ein Anion steht, z.B. Chlorid, Bromid und Methosulfat, auf die künstlich gefärbten Haare aufgebracht und mit Wasser ausgespült wird.

2. Verfahren nach einem der vorhergehenden Ansprüche, **dadurch gekennzeichnet, dass** die Reinigungszusammensetzung zusätzlich nichtionische schaumbildende Tenside aufweist.

3. Verfahren nach einem der vorhergehenden Ansprüche, **dadurch gekennzeichnet, dass** die Reinigungszusammensetzung anionische, nichtionische und amphotere schaumbildende Tenside aufweist.

4. Verfahren nach einem der vorhergehenden Ansprüche, **dadurch gekennzeichnet, dass** die anionischen und nichtionischen Tenside in der Reinigungszusammensetzung in einem Gewichtsverhältnis anionischer zu nichtionischer Tenside im Bereich von 10:1 bis 1:1 enthalten sind.

5. Verfahren nach einem der vorhergehenden Ansprüche, **dadurch gekennzeichnet, dass** die anionischen und amphoteren Tenside in der Reinigungszusammensetzung in einem Gewichtsverhältnis anionischer zu amphoterer Tenside im Bereich von 10:1 bis 1:1 enthalten sind.

6. Verfahren nach einem der vorhergehenden Ansprüche, **dadurch gekennzeichnet, dass** die Reinigungszusammensetzung Alkylpolyglucosid und/oder ethoxylierten Fettalkohol als nichtionische schaumbildende Tenside aufweist.

7. Verfahren nach einem der vorhergehenden Ansprüche, **dadurch gekennzeichnet, dass** die Reinigungszusammensetzung Alkylbetaine, Alkylamidobetaine und/oder Hydroxysulfobetaine als amphotere schaumbildende Tenside aufweist.

8. Verfahren nach einem der vorhergehenden Ansprüche, **dadurch gekennzeichnet, dass** die Reinigungszusammensetzung mindestens ein kationisches Tensid aufweist, ausgewählt aus Behentrimoniumchlorid, Behentrimoniummethosulphat, Ceteartrimoniumchlorid, Cetrimoniumchlorid, Cetrimoniumbromid, Cetrimoniummethosulphat, Cocotrimoniumchlorid, Cocotrimoniumbromid, Laurtrimoniumchlorid, Laurtrimoniumbromid, Myrtrimoniumchlorid, Steartrimoniumchlorid, Steartrimoniumchlorid und Sojatrimoniumchlorid.

9. Verfahren nach einem der vorhergehenden Ansprüche, **dadurch gekennzeichnet, dass** die Reinigungszusammensetzung mindestens ein kationisches Tensid in einer Konzentration im Bereich von 0,1 Gewichts-% bis 5 Gewichts-% aufweist, bezogen auf die Gesamtzusammensetzung.

10. Verfahren nach einem der vorhergehenden Ansprüche, **dadurch gekennzeichnet, dass** die Reinigungszusammensetzung mindestens ein Haarkonditionierungsmittel aufweist, vorzugsweise ausgewählt aus kationischen Polymeren.

11. Verfahren nach einem der vorhergehenden Ansprüche, **dadurch gekennzeichnet, dass** die Reinigungszusammensetzung eine oder mehrere Substanzen aufweist, ausgewählt aus organischen Lösungsmitteln, Fettalkoholen, Perlglanzmitteln, Löslichkeitsvermittlern, UV-Filtern, Polyolen, Maskierungsmitteln, natürlichen Pflanzenextrakten, Ubichinonen, Direktfarbstoffen und Verdickungsmitteln.

12. Verwendung mindestens eines kationischen Tensids der allgemeinen Struktur 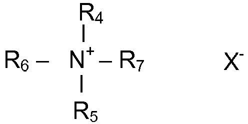 wobei R₄ für eine gesättigte oder ungesättigte, verzweigte oder lineare Alkylkette mit 8 bis 22 C-Atomen steht, R₅, R₆ und R₇ für H oder eine gesättigte oder ungesättigte, verzweigte oder lineare Alkylkette mit 1 bis 4 C-Atomen oder eine gesättigte oder ungesättigte, verzweigte oder lineare C₂₋₄-Alkylkette mit einem oder mehreren Substituenten, vorzugsweise einem Hydroxy-Substituenten, stehen und X typischerweise für ein Anion steht, z.B. Chlorid, Bromid und Methosulfat, in einer Reinigungszusammensetzung, welche 5 Gewichts-% bis 50 Gewichts-% schaumbildendes Tensid aufweist, bezogen auf die Gesamtzusammensetzung, wobei die Zusammensetzung mindestens ein Alkylethersulfat-Tensid und mindestens ein Aminosäure-Tensid als anionische schäumende Tenside aufweist, zur Verringerung des Auswaschens der Farbe aus künstlich gefärbten Haaren.

## Revendications

1. Procédé de traitement des cheveux artificiellement colorés, **caractérisé en ce qu'**une composition de nettoyage comprenant 5 à 50 % en poids, calculés par rapport à la composition totale, de tensioactifs moussants, où la composition comprend au moins un tensioactif de type sulfate d'éther d'alkyle et au moins un tensioactif de type acide aminé an tant que tensioactifs moussants et au moins un tensioactif cationique selon la structure générale 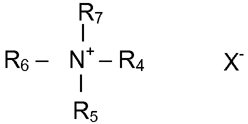 où R₄ est une chaîne alkyle saturée ou non saturée, linéaire ou ramifiée, avec 8 à 22 atomes C, R₅, R₆ et R₇ sont un atome H ou une chaîne alkyle saturée ou non saturée, linéaire ou ramifiée avec de 1 à 4 atomes C, ou une chaîne alkyle saturée ou non saturée, linéaire ou ramifiée en C2 à C4 avec un ou plusieurs substituants, de préférence, un substituant de type hydroxyle et X est généralement un anion tel qu'un chlorure, un bromure et un méthosulfate, est appliquée sur les cheveux artificiellement colorés et est éliminée par un rinçage à l'eau.

2. Procédé selon l'une quelconque des revendications précédentes, **caractérisé en ce que** la composition de nettoyage comprend en outre des tensioactifs moussants non ioniques.

3. Procédé selon l'une quelconque des revendications précédentes, **caractérisé en ce que** la composition de nettoyage comprend des tensioactifs moussants anioniques, non ioniques et amphotères.

4. Procédé selon l'une quelconque des revendications précédentes, **caractérisé en ce que** les tensioactifs anioniques et non ioniques sont compris dans la composition de nettoyage dans un ratio pondéral entre les tensioactifs anioniques et les tensioactifs non ioniques dans une plage de 10 : 1 à 1 : 1.

5. Procédé selon l'une quelconque des revendications précédentes, **caractérisé en ce que** les tensioactifs anioniques et amphotères sont compris dans un ratio pondéral entre les tensioactifs anioniques et les tensioactifs amphotères dans une plage de 10 : 1 à 1 : 1.

6. Procédé selon l'une quelconque des revendications précédentes, **caractérisé en ce que** la composition de nettoyage comprend un polyglucoside d'alkyle et/ou un alcool gras éthoxylé en tant que tensioactifs moussants non ioniques.

7. Procédé selon l'une quelconque des revendications précédentes, **caractérisé en ce que** la composition de nettoyage comprend des alkyl bétaïnes, des alkyl amino bétaïnes et/ou des hydroxy sulfobétaïnes en tant que tensioactifs moussants amphotères.

8. Procédé selon l'une quelconque des revendications précédentes, **caractérisé en ce que** la composition de nettoyage comprend au moins un tensioactif cationique choisi parmi le chlorure de béhentrimonium, le méthosulfate de béhentrimonium, le chlorure de cétéartrimonium, le chlorure de cétrimonium, le bromure de cétrimonium, le méthosulfate de cétrimonium, le chlorure de cocotrimonium, le bromure de cocotrimonium, le chlorure de laurtrimonium, le bromure de laurtrimonium, le chlorure de myrtrimonium, le chlorure de stéartrimonium, le chlorure de stéartrimonium et le chlorure de soytrimonium.

9. Procédé selon l'une quelconque des revendications précédentes, **caractérisé en ce que** la composition de nettoyage comprend au moins un tensioactif cationique à une concentration dans une plage de 0,1 à 5 % en poids calculée par rapport à la composition totale.

10. Procédé selon l'une quelconque des revendications précédentes, **caractérisé en ce que** la composition de nettoyage comprend au moins un agent de conditionnement des cheveux, choisi de préférence parmi les polymères cationiques.

11. Procédé selon l'une quelconque des revendications précédentes, **caractérisé en ce que** la composition de nettoyage comprend une ou plusieurs des substances choisies parmi les solvants organiques, les alcools gras, les agents apportant un effet nacré, les agents de solubilisation, les filtres UV, les polyols, les agents séquestrants, les extraits de plantes naturelles, les ubiquinones, les colorants directs et les agents épaississants.

12. Utilisation d'au moins un tensioactif cationique selon la structure générale 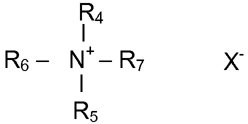 où R₄ est une chaîne alkyle saturée ou non saturée, linéaire ou ramifiée, avec de 8 à 22 atomes C, R₅, R₆ et R₇ sont un atome H ou une chaîne alkyle saturée ou non saturée, linéaire ou ramifiée, avec de 1 à 4 atomes C ou une chaîne alkyle saturée ou non saturée, linéaire ou ramifiée, en C2 à C4 avec un ou plusieurs substituants, de préférence un substituant de type hydroxyle, et X est généralement un anion tel qu'un chlorure, un bromure et un méthosulfate, dans une composition de nettoyage comprenant de 5 à 50 % en poids, calculés par rapport à la composition totale, de tensioactifs moussants, où la composition comprend au moins un tensioactif de type sulfate d'éther d'alkyle et au moins un tensioactif de type acide aminé en tant que tensioactifs moussants anioniques pour réduire la décoloration des cheveux artificiellement colorés.
